# EUROPEAN PATENT SPECIFICATION

(11) **EP 0 366 279 B1**
(45) Date of publication and mention of the grant of the patent: **21.09.1994**
(21) Application number: 89310016.4
(22) Date of filing: 29.09.1989
(51) Int. Cl.: A61K 31/557

(54) **Ocular hypotensive agents**
Okulare hypotensive Mittel
Agents oculaires hypotenseurs

(30) Priority: 01.10.1988 JP 248720/88; 01.10.1988 JP 248721/88
(43) Date of publication of application: 02.05.1990
(60) Divisional application: 93202691.7
(73) Proprietor: R-TECH UENO, LTD., Osaka-shi, Osaka-fu (JP)
(72) Inventor: Ueno, Ryuzo, Nishinomiya-shi Hyogo-ken (JP); Ueno, Ryuji, Nishinomiya-shi Hyogo-ken (JP); Oda, Tomio, Sanda-shi Hyogo-ken (JP)
(74) Representative: Atkinson, Peter Birch

(56) References cited:
- EP-A- 0 093 380
- EP-A- 0 253 094
- EP-A- 0 308 135
- EP-A- 0 330 511
- FR-A- 2 110 335
- INVESTIGATIVE OPHTHALMOLOGY & VISUAL SCIENCE, December 1977; C.B. CAMRAS et al.: "Reduction of intraocular pressure by prostaglandins applied topically to the eyes of conscious rabbits"

## Description

### Background of the Invention

The present invention relates to ocular hypotensive agents or agents used for treatment of glaucoma which contain a prostaglandins, in which the carbon atom of the 20-position is substituted with a hydrocarbon group.

Prostaglandins (hereinafter referred to as PGs) is the name given to the group of fatty acids which show various physiological activities and which are contained in human and animal tissues and organs. PGs essentially contain the prostanoic acid skeleton of the following formula: 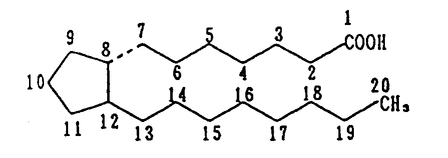
Some synthetic products may also contain the above skeleton with some modification.

PGs are classified into several types according to their five-membered ring, for example, 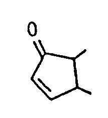
prostaglandins of the A series (PGAs): 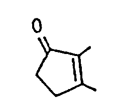
Prostaglandins of the B series (PGBs): 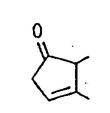
Prostaglandins of the C series (PGCs): 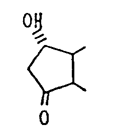
Prostaglandins of the D series (PGDs): 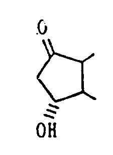
Prostaglandins of the E series (PGEs): 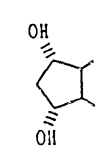
Prostaglandins of the F series (PGFs): and 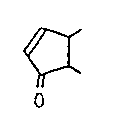
Prostaglandins of the J series (PGJs):
Further, they are classified into PG₁s containing 5,6-single bond: 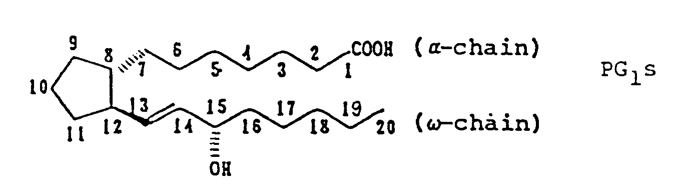
PG₂s containing 5,6-double bond: 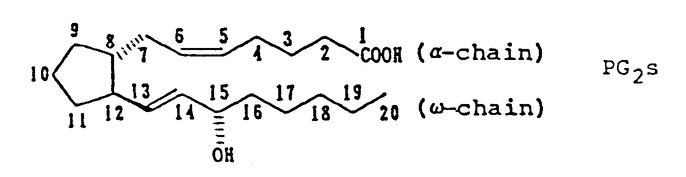
and PG₃s containing 5,6-and 17,18-double bonds: 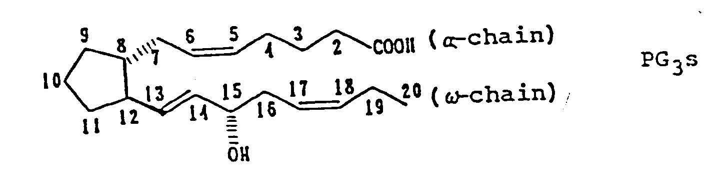
PGs are known to have various pharmacological and physiological activities, for example, vasodilation, induction of inflammation, platelet aggregation, contraction of uterine muscle and enteron contraction. However, PGs also possesses various other activities. Therefore there are some problems in their use as medicines. That is, when PGs are administered to obtain a single pharmaceutical activity, they often exhibit other activities as side effects. Accordingly, the investigations of PGs as a medicine have aimed to enhance their the main pharmaceutical activity. However, these investigations have been insufficient.

Among PGs, for example, PGAs, PGDs, PGEs, PGFs are known to possess ocular hypotensive potency.

For example, it is disclosed in Japanese Patent Application KOKAI No. 1418/1984 (claiming a priority based on U.S. Ser. No. 374165(1982) by Laszlo Z. Bite) that PGF₂ has a high ocular hypotensive activity, and that 15-keto-PGF₂ also has the activity though to a much reduced extent. Further Japanese Patent Application KOKAI No. 66122/1988 (claiming priorities based on three U.S. Ser. Nos. 839056 (1986), 892387(1986) and 022046 (1987)) disclose that PGA, PGB and PGC can be used for a treatment of glaucoma.

However, when these PGs are applied topically to rabbit eyes, they produce a transient ocular hypertensive response. Pronounced conjunctival and iridal hyperemia, and further side effects such as lacrimation, eye mucus and lid closure are also observed. Accordingly, there are some problems when PGs are used as remedies for glaucoma or ocular hypotensive agents. According to the present invention there is provided the use of a composition comprising an amount of 15-hydroxy-prostaglandin of type A, E or F having one or more hydrocarbon substituent(s) at the 20-position, physiologically acceptable salts or esters thereof effective as an ocular hypotensive agent and a pharmaceutically acceptable carrier for the manufacture of a medicament for the treatment of ocular hypertension.

It has been found that PGs as defined in the preceding paragraph (ie. PGs in which the carbon atom of the 20-position is substituted with a hydrocarbon group (referred to as 20-substituted PGs hereinafter)) cause intraocular pressure reduction without any transient ocular hypertensive response that PGs usually show. Further, they possess enhanced ocular hypotensive potency, and exhibit ocular hypotensive effect without transient ocular hypertensive response, and with absolutely no or extremely reduced side effects such as hyperemia of conjunctiva or of iris, dacryops, lema, closed eye and the like.

The present invention provides an ocular hypotensive composition or a composition for treatment of glaucoma, which contains PGs, in which the carbon atom of the 20-position is substituted with a hydrocarbon group (ie. 20-substituted-PGs) as active ingredients.

In this description, 20-substituted-PGs. are named as follows, Viz the carbons constituting the α-chain, ω-chain and 5-membered ring are numbered according to the basic skeleton as follows: 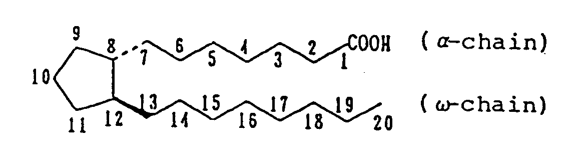
That is, in the basic skeleton, the constituent carbon atoms are numbered in such a way that the carboxylic acid carbon atom is C-1, the α-chain contains C-2 - C-7 (the number increasing toward the ring), the five-membered ring contains C-8 - C-12, and the ω-chain contains C-13 - C-20. When there are fewer than 7 carbons in the α-chain, the numbers of the carbons following C-2 should simply be eliminated from 2 to 7 in this order, and when more than 7, the compound is named such that the 'increase' is named as a substituent on the carbon at the 2 position. When the ω-chain contains fewer carbon atoms, they should be numbered correspondingly smaller than 20 and when more than 8, the carbon atoms at the 21 position and thereafter should be regarded as a substituent. As configuration, it is considered according to that of the above essential skeleton unless otherwise described.

For example, PGE and PGF mean compounds having a hydroxyl group at the 9 and/or 11 positions. In the present invention, PGs include compounds having a group of the hydroxyl group on the 9 and/or 11 positions, such compounds being named as 9-dehydroxy-9-substituted or 11-dehydroxy-11-substituted compounds.

20-substituted-PGs used in the present invention may be 20-substituted PG₁s containing a 5,6-single bond; 20-substituted-PG₂s or containing a 5,6-double bond; or 20 substituted-PG₃s containing both 5,6- and 17,18-double bonds.

The typical examples of the PGs used in the present invention are shown below:
20-substituted -PGA₁s,
20-substituted -PGA₂s,
20-substituted -PGA₃s,
20-substituted or -PGE₁s,
20-substituted or -PGE₂s,
20-substituted or -PGE₃s,
20-substituted or -PGF₁s,
20-substituted or -PGF₂s,
20-substituted or -PGF₃s,
Derivatives, esters or salts of these PGs may be used, too.

These 20-substituted-PGs show strong ocular hypotensive potency without showing transient ocular hypertensive response. Further, side effects such as pronounced conjunctival or iridal hyperemia, lacrimation and lid closure are either not present or are extremely reduced. Accordingly, these 20-substituted PGs are extremely effective as ocular hypotensive agents. Further, depending on such ocular hypotensive effect, they may be used for glaucoma therapy.

In the present invention, the ocular hypotensive effect of 20-substituted PGs or may be especially remarkable in prostaglandins of the general formula: 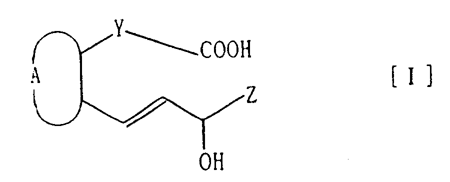
[wherein, A is 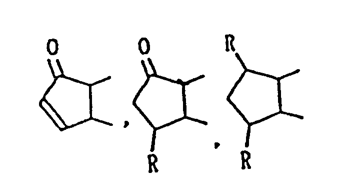
(in which R is hydroxyl, hydroxyalkyl or alkyl);
Y is a saturated or unsaturated C₂₋₆ hydrocarbon chain (a part of the carbon atoms constituting the hydrocarbon chain may form a carbonyl group, and the hydrocarbon chain may be substituted with one or more atoms or groups);
Z is a C₆₋₁₀ saturated or unsaturated aliphatic, alicyclic, aralkyl or aromatic hydrocarbon (the hydrocarbon may be substitued with one or more atoms or groups)) or physiologically acceptable salts derived from the general formula [I] or those having an esterified carboxyl group.

Further preferred compounds for use in accordance with the invention are represented by the formula (II): 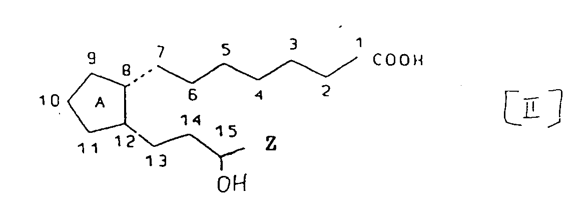
wherein A is 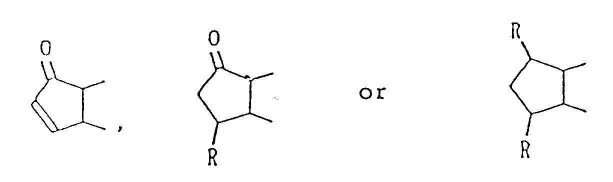
in which R is hydroxyl, hydroxyalkyl or alkyl; and Z is a C₇ - C₁₀ saturated or unsaturated aliphatic, alicyclic, aralkyl or aromatic hydrocarbon group; or physiologically acceptable salts or esters thereof.

A saturated or unsaturated C₂₋₆ hydrocarbon chain Y includes a straight hydrocarbon chain such as an alkyl, alkenyl, and alkynyl. Especially, a hydrocarbon chain with 6 carbons is preferred.

Examples of an unsaturated hydrocarbon chain Y include, for example, PGs in which carbons at the 2-3 positions or the 5-6 positions are joined by an unsaturated bond.

Some of the carbons forming the hydrocarbon chain Y may form a carbonyl group. A typical example includes 6-keto-PG₁s wherein the carbon at the 6 position constitutes a carbonyl group.

The hydrocarbon chain Y may be substituted with one or more atoms or groups. Such atoms or groups include, for example, a halogen atom such as a fluorine, chlorine or bromine atom; an alkyl group such as methyl, ethyl; a hydroxyl group. A typical example is one or more alkyl groups at the 3-carbon atom.

The hydrocarbon group Z is a C₆-C₁₀ alkyl group, preferably C₇-C₉ alkyl group, which may have one or more branch(es). Preferable examples of the hydrocarbon group Z is C₆ alkyl group, C₇ alkyl group, C₈ alkyl group or C₉ alkyl group. Most, preferably Z is an alkyl group having no branch.

The hydrocarbon Z may be substituted with one or more atoms or groups. Such atoms or groups include a halogen atom such as a fluorine, chlorine or bromine atom; an alkyl group such as a methyl, ethyl, isopropyl or isopropenyl group; an alkoxy group such as a methoxy or ethoxy group; a hydroxyl group; a phenyl group; a phenoxy group and the like. The position of the substituent atom(s) or group(s) is not limited, but typically, they may be at 16, 17, 19 and/or 20 position in the ω-chain. Particularly preferred are compounds having one or two of the same or different atoms at the 16 position, for example, a halogen atom such as a fluorine atom or a substituent, for example, an alkyl group such as a methyl, ethyl, hydroxyl phenyl which may contain one or more substituents, benzyl, phenoxy, or cycloalkyl group such as a cyclopentyl or cyclohexyl group which contains the 16 position as a constituent; compounds having an alkyl group such as methyl at the 17 or 19 position; and compounds having an alkyl group such as a methyl, ethyl, isopropyl, isopropenyl or alkoxy group such as a methoxy, ethoxy or propoxy group at the 20 position.

PGs may include the compounds PGE, PGF which contain a hydroxyl group at the 9 and/or 11 position. In the present specification, PGs further include the compounds having a hydroxyalkyl or alkyl group instead of the hydroxyl group at the 9 and/or 11 position. Accordingly, the 20-substituted-PGs of the present invention include the compound of the general formula [I], wherein R is a hydroxyl, hydroxyalkyl or alkyl group. Such a hydroxyalkyl group is preferably a hydroxymethyl or 1-hydroxyethyl, 2-hydroxyethyl or 1-methyl-1-hydroxyethyl group. As the alkyl group, a lower alkyl group, especially a methyl or ethyl group is preferred.

The configuration of R for the carbon at the 9 and/or 11 position may be α, β or mixture thereof.

PGs of the present invention may be salts or esters. Such salts include physiologically acceptable salts, for example, those of an alkali metal such as sodium, potassium; those of an alkaline earth metal such as calcium, magnesium; those of physiologically acceptable an ammonium or amine salt such as ammonia, methylamine, dimethylamine, cyclopentylamine, benzylamine, piperidine, monoethanolamine, diethanolamine, monomethylmonoethanolamine, tromethamine, lysine, tetralkylammonium salt and the like. Such an ester includes, for a example, methyl, ethyl, propyl, butyl, isopropyl, t-butyl, 2-ethylhexyl, straight or branched-chain alkyl ester which may contain an unsaturated bond; for example, ester having an alicyclic group such as a cyclopropyl, cyclopentyl or cyclohexyl group; an ester containing an aromatic group such as a benzyl or phenyl group (wherein the aromatic group may contain one or more substituents); a hydroxyalkyl or alkoxyalkyl ester such as a hydroxyethyl, hydroxyisopropyl, polyhydroxyisopropyl, methoxyethyl, ethoxyethyl or methoxyisopropyl group; an alkylsilyl ester e.g., a trimethylsilyl or triethylsilyl ester; a tetrahydropyranyl ester.

Preferred esters include, for example, a straight or branched lower alkyl ester such as a methyl, ethyl, propyl, n-butyl, isopropyl or t-butyl ester; or a benzyl ester; a hydroxyalkyl ester such as a hydroxyethyl or hydroxyisopropyl ester.

The carboxyl group at the 1 position of 20-substituted-PGs of the present invention may be any of the above described groups. Among them, esters, especially the C₁₋₄ alkyl ester, especially isopropyl ester are preferred.

20-substituted-PGs or of the present invention may include the isomers of the above compounds. Examples of such isomers include keto-hemiacetal tautomers between the C₆-carbonyl and C₉-hydroxyl; optical isomers; and geometrical isomers.

The mixture of the isomers, for example, racemic mixtures and mixtures of tautomers of hydroxyl compound and hemiacetals may show similar effect as that shown by the respective compound.

In the present invention, especially preferred 20-substituted-PGs contain a 5,6-single or double bond, or a carbonyl group at the C-6 position atom. Other preferred compounds are 20-substituted PGs wherein the carbon atom at the 16 position may be substituted with a halogen atom or an alkyl group.

Particularly, the compounds having a C₁₋₄ alkyl, (for example, a methyl, ethyl, propyl or butyl group) at the 20 position (ie having a prolonged ω-chain) show enhanced ocular hypotensive effect with little side effects such as hyperemia. Accordingly, such compounds are preferred.

In the present invention, PGs are named according to the prostanoic acid skeleton. If named according to IUPAC, for example, PGE₁ corresponds to 7-[(1R,2R,3R)-3-hydroxy-2-[(E)-(3S)-3-hydroxy-1-octenyl]-5-oxo-cyclopentyl]-heptanoic acid; PGE₂, (Z)-7-[(1R,2R,3R)-3-hydroxy-2-[(E)-(3S)-3-hydroxy-1-octenyl]-5-oxo-cyclopentyl]-hept-5-enoic acid; 20-ethyl-PGE₁ is 7-{(1R,2R,3R)-3-hydroxy-2-[(E)-(3S)-3-hydroxy-1-decenyl]-5-oxo-cyclopentyl}-heptanoic acid. PGF₁α corresponds to 7-[(1R,2R,3R,5S)-3,5-dihydroxy-2-[(E)-(3S)-3-hydroxy-1-octenyl]-cyclopentyl]-heptanoic acid; PGF₂α , (Z)-7-[(1R,2R,3R,5S)-3,5-dihydroxy-2-[(E)-(3S)-3-hydroxy-1-octenyl]-cyclopentyl]-5-heptenoic acid; therefore, 20-ethyl-PGF₁α is 7-[(1R,2R,3R,5S)-3,5-dihydroxy-2{(E)-(3S)-3-hydroxy-1-decenyl}-cyclopentyl]-heptanoic acid. PGF₂α is (Z)-7-[(1R,2R,3R,5S)-3,5-dihydroxy-2{(E)-(3S)-3-hydroxy-1-octenyl}-cyclopentyl]-5-heptanoic acid Other PGs may also be named in the same way.

In the process for preparing 20-substituted-PGs: A commercially available (-)-Corey lactone, which is used as a starting material, is subjected to Collins oxidation to give an aldehyde. The aldehyde is allowed to react with dimethyl (2-oxoalkyl)phosphonate anion which has a desirable length of alkyl group to give an α,β-unsaturated ketone (in order to obtain 20-methyl-PGs dimethyl(2-oxooctyl)phosphonate anion is used).

The obtained α,β-unsaturated ketone was reduced using sodium borohydride to give α,β-unsaturated hydroxyl compound, the hydroxyl group of which is protected with THP (see Synthetic Chart I). The precursor of 20-substituted-PGs introduced with ω-chain obtained from the above process can be converted to 20-substituted-PGs according to a general process for production of PGs.

PGs containing a methyl group instead of a hydroxy group at the 11 position may be obtained as follows: PGA obtained by Jones oxidation of the hydroxy group at the 9 position of the 11-tosylate is allowed to react with a dimethyl copper complex to give 11-dehydroxy-11-methyl-PGE. Alternatively, an alcohol obtained after elimination of p-phenylbenzoyl group is converted to a tosylate. An unsaturated lactone obtained by DBU treatment of the tosylate is converted to a lactol. After introduction of an α -chain using Wittig reaction, the resulting alcohol (9 position) is oxidized to give PGA. PGA is allowed to react with dimethyl copper complex to give 11-dehydroxy-11-methyl-PGE. The resultant is reduced using sodium borohydride and the like to give 11-dehydroxy-11-methyl-PGF.

PGs containing a hydroxymethyl group instead of a hydroxyl group at the 11 position is obtained as follow: 11-dehydroxy-11-hydroxymethyl-PGE is obtained by a benzophenone-sensitized photoaddition of methanol to PGAs. The resultant is, for example, reduced using sodium borohydride to give 11-dehydroxy-11-hydroxymethyl-PGFs.

16-Fluoro-PGs may be obtained using a dimethyl (3-fluoro-2-oxoalkyl)phosphonate anion in the preparation of an α,β-unsaturated ketone. Similarly, 19-methyl-PGs may be obtained using a dimethyl (6-methyl-2-oxoalkyl)phosphonate anion.

The preparations in the present invention are not construed to be limited to them, and suitable means for protection, oxidation, reduction and the like may be employed.

20-substituted-PGs of the present invention can be used as remedies for animal and human, and, in general, used for systemic or local application by oral administration, intravenous injection, subcutaneous injection, suppository, collyrium, oculentum and the like. The dosage varies depending on animals, human, age, weight, conditions, therapeutic effect, administration route, treatment time and the like.

The solid composition for oral administration of the present invention includes tablets, preparations, granules and the like. In such a solid composition, one or more active ingredients may be mixed with at least one inactive diluent, for example, lactose, mannitol, glucose, hydroxypropyl cellulose, microcrystalline cellulose, starch, polyvinyl pyrrolidone, magnesium aluminate metasilicate and the like. According to the usual work-up, the composition may contain additives other than an inactive diluent, for example, a lubricant such as magnesium stearate; disintegrant such as fibrous calcium gluconate; a stabilizer such as etherified cyclodextrin, for example, α,β- or γ-cyclodextrin, dimethyl-α-, dimethyl-β-, trimethyl-β- or hydroxypropyl-β-cyclodextrin, branched cyclodextrin such as glucosyl-, maltosyl-cyclodextrin, formylated cyclodextrin, cyclodextrin containing sulfur, mitthoprotol, phospholipid and the like. When the above cyclodextrins are used, an inclusion compound with cyclodextrins may be sometimes formed to enhance stability. Alternatively, a phospholipid may be sometimes used to form a liposome, resulting in enhanced stability.

Tablets or pills may be coated with film soluble materials in the stomach or intestine such as sugar, gelatin, hydroxypropyl cellulose, hydroxypropylmethyl cellulose phthalate and the like, or with more than two layers. Further, they may be formed as capsules with absorbable substances such as gelatin.

A liquid composition for oral administration may contain a pharmaceutically acceptable emulsion, solution, suspension, syrup, elixir as well as a generally used inactive diluent, for example, purified water, ethanol and the like. Such a composition may contain, in addition to the inactive diluent, adjuvants such as wetting agents and suspensions, sweetening agents, flavoring agents, preservatives and the like.

Other compositions for oral administration include a spray formulated by known method, which may contain one or more active ingredients.

Injection for parenteral administration according to the present invention includes a steril, aqueous or nonaqueous solution, suspension, emulsion and the like.

A diluent for such an aqueous solution and suspension includes, for example, injectable distilled water, physiological saline and Ringer's solution.

A diluent for non-aqueous solution and suspension includes, for example, propylene glycol, polyethylene glycol, vegetable oil such as olive oil, alcohols such as ethanol, polysorbate and the like. Such a composition may contain adjuvants such as preservatives, wetting agents, emulsifiers, dispersants, stabilizers and the like. These are sterilized, for example, by filtration through a bacteria-holding filter, compounding with germicides, gas sterilization or radio-sterilization. These may be used by preparing a sterile solid composition and dissolving in sterile water or sterile solvent for injection before use.

The collyrium according to the present invention may include a sterile aqueous or non-aqueous solution, suspension and the like. The diluent for such an aqueous solution or suspension includes, for example, distilled water or a physiological saline. The diluent for the non-aqueous solution or suspension may include an edible oil, liquid paraffin, mineral oil, propylene glycol, p-octyldodecanol and the like. Further, in order to make isotonic to tears, isotonic agents such as sodium chloride, benzalkonium chloride, phedrine chloride, procaine chloride, chloram phenicol, sodium citrate, or in order to maintain the pH value constant, buffer such as a borate or phosphate buffer may be used. Moreover, stabilizers such as sodium sulfite, sodium carbonate, EDTA, propylene glycol; thickening agents such as glycerin, carboxymethyl cellulose, carboxyvinyl polymer; diluents such as polysorbate, macrogols, alminum monostearate; preservatives such as paraben, benzyl alcohol, sorbic acid; and further resolvents, vehicles may be compounded. These may be sterilized, for example, by the filtration through a bacteria-holding filter or heat sterilization. In the preparation of collyrium, pH value and ion strength of the agent are especially important, and they may be optionally adjusted to the optimal value depending on the types and amounts of other active ingredients or additives used.

The oculentum according to the present invention may contain vaseline, selen 50, plastibase, macrogols as a base, and surfactant such as polysorbate, Tween, purified lanolin, jelly such as carboxymethyl cellulose, methylcellulose, carboxyvinyl polymer to enhance hydrophilism.

The ocular hypotensive agent of the present invention may be used as a remedy for glaucoma utilizing its ocular hypotensive potency. When used as the remedie for treatment of glaucoma, the present agents may be compounded with the conventional cholinergic ocular hypotensive agent (e.g., pilocarpine, carbachol, which possesses excellent miotic activity) anticholinesterases (e.g., demecarium, D.F.P., echothiophate), physostigmine salicylate, pilocarpine hydrochloride as miotics, mannitol, glycerin, isosorbide as hyperosmotic agent for intravenous injection, chlorobutanol, benzalkonium chloride, propylparabene, methylparaben, ethylparaben, butylparaben as preservatives for collyrium, penicillin, sulfonamide, chloramphenicol, cortisone, chlorpheniramine for prevention and treatment of other inflammation.

The present invention will be illustrated in the following examples.

### EXAMPLE 1 (Preparations of 20-substituted-PGs; see Synthetic Chart I)

1 - 1) Preparation of (1S,5R,6R,7R)-6-(3-oxo-(E)-1-decenyl)-7-(4-phenylbenzoyloxy)-2-oxabicyclo[3.3.0]octane-3-one (3):
   Commercially available (-)-Corey lactone (1) (7.0 g) was subjected to Collins oxidation in dichloromethane to give aldehyde (2). The aldehyde (2) was reacted with anion obtained from dimethyl (2-oxononyl)phosphonate (4.97 g) to give the title compound (3). Yield: 5.71 g
1 - 2) Preparation of (1S,5R,6R,7R)-6-[3(S)-hydroxy-(E)-1-decenyl]-7-(4-phenylbenzoyloxy)-2-oxabicyclo[3.3.0]octane-3-one (4a):
   The unsaturated ketone (3) (3.03 g) was dissolved into a mixed solvent of methanol (60 ml) and THF (7 ml). Into the obtained solution cerium (III) chloride ·7H₂O (2.38 g) was added at -20°C, and stirred at the same temperature for 10 minutes, into which sodium borohydride (0.25 g) was added and stirred for 5 minutes. A crude compound obtained by a usual work-up was purified using a column chromatography to give the title compound (4a) as a compound having a lower polarity and the 3R-hydroxy compound (4b) of the title compound (4a) as a compound having a higher polarity. Yield: 4.58 g (4a:3S-hydroxy compound)
1 - 3) Preparation of (1S,5R,6R,7R)-7-(4-phenylbenzoyloxy)-6-[3(S)-(2-tetrahydropyranyloxy)-(E)-1-decenyl]-2-oxabicyclo[3.3.0]octane-3-one (5):
   The 3S-hydroxy compound (4a) (4.58 g) was dissolved into methylene chloride (100 ml), and reacted with dihydropyran in the presence of a catalytic amount of p-toluenesulfonic acid-1H₂O. After a usual work-up, the reaction mixture was column-chromatographed to give the title compound (5). Yield: 5.03 g
1 - 4) Preparation of (1S,5R,6R,7R)-7-hydroxy-6-[3(S)-(2-tetrahydropyranyloxy)-(E)-1-decenyl]-2-oxabicyclo[3.3.0]octane-3-one (6):
   The tetrahydropyranyl ether (5) (5.03 g) was dissolved into a dry methanol (300 ml), into which potassium carbonate (1.49 g) was added, and stirred at room temperature for 6 hours. A crude compound obtained by a usual work-up was column-chromatographed to give the title compound (6). Yield: 3.25 g
1 - 5) Preparation of (1S,2RS,5R,6R,7R)-3,7-dihydroxy-6-[3(S)-(2-tetrahydropyranyloxy)-(E)-1-decenyl]-2-oxabicyclo[3.3.0]octane (7):
   Alcohol (6) (2.00 g) was reduced in dry toluene (40 ml) using DIBAL-H at -70°C. The lactol (7) was obtained by a usual work up.
1 - 6) Preparation of 20-ethyl-15S-(2-tetrahydropyranyloxy)-PGE₂α(8):
   In dimethyl sulfoxide the lactol (7) was reacted with an ylide prepared from (4carboxybutyl)triphenylphosphonium bromide (8.33 g). The title compound(s) was obtained by a usual work-up.
1 - 7) Preparation of 20-ethyl-15S-(2-tetrahydropyranyloxy)-PGF₂α isopropyl ester (9):
   Carboxylic acid (8) was dissolved in acetonitrile (50 ml), into which DBU (1.1 g) and isopropyl iodide (2.5 g) were added. The mixture was stirred at 45 °C for 5 hours. A crude compound obtained by a usual work-up was column-chromatographed to give the title compound (9). Yield: 1.00 g
1 - 8) Preparation of 11R-(t-butyldimethylsiloxy)-20-ethyl-15S-(2-tetrahydropyranyloxy)-PGF₂α isopropyl ester (10):
   The isopropyl ester (9) (0.37 g) was dissolved into dry DMF (3 ml), and reacted with imidazole (0.059 g) and t-butyldimethylsilyl chloride (0.132 g). A crude compound obtained by a usual work-up was purified by a column chromatography to give the title compound (10). Yield: 0.26 g
1 - 9) Preparation of 11R-(t-butyldimethylsiloxy)-20-ethyl-15S-(2-tetrahydropyranyloxy)-PGE₂ isopropyl ester (11):
   The 11-silyl ether (10) (0.303 g) in acetone (20 ml) was oxidized using Jones reagent at -40 °C to give the title compound (11). Yield: 0.27 g
1 - 10) Preparation of 20-ethyl-PGE₂ isopropyl ester (12) and 20-ethyl-PGA₂ isopropyl ester (13):
   11R-(t-butyldimethylsiloxy)-20-ethyl-15S-(2-tetrahydropyranyloxy)-PGE₂ isopropyl ester (11) (0.27 g) was stirred in a mixed solvent of acetic acid/water (7/1) (20 ml) at 65 °C for 15 hours. A crude product obtained by a concentration under reduced pressure was column-chromatographed to give the title compound (12) as a colorless oily compound (higher polarity) and the title compound (13) as a colorless oily compound (lower polarity). Yield of 20-ethyl-PGE₂ isopropyl ester (12) and 20-ethyl-PGA₂ isopropyl ester (13) are 0.040 g and 0.080 g respectively.
1 - 11) Preparation of 20-ethyl-PGF₂α isopropyl ester (14):
   The 20-ethyl-15S-(2-tetrahydropyranyloxy)-PGF₂α isopropyl ester (9) (0.60 g) was dissolved in a mixed solvent of acetic acid/water/THF (3/1/1) (20 ml), and maintained at 40 - 45 °C for 3 hours. A crude product obtained by the concentration under reduced pressure was column-chromatographed to give the title compound (14) as a colorless solid material. Yield: 0.023 g

The ¹H NMR and Mass spectra of the 20-ethyl-PGF₂α isopropyl ester are shown hereinafter:
¹H NMR(CDCl₃) δ : 0.86(3H,t,J=6Hz), 1.18(6H,d,J=7Hz), 1.10 - 2.60(26H,m), 3.17(1H,m), 3.70 - 4.23(3H,m), 4.95(1H,hept,J=7Hz), 5.20 - 5.60(4H,m)
Mass(EI) m/z 424(M⁺), 406(M⁺-H₂O), 388(M⁺-2H₂O), 370(M⁺-3H₂O)
The ¹H NMR and Mass spectra of the 20-ethyl-PGE₂ isopropyl ester are shown hereinafter:
¹H NMR(CDCl₂) δ : 0.88(3H,t,J=5Hz), 1.23(6H,d,J=7Hz), 1.04 - 2.90(25H,m), 3.11(1H,m), 3.85 - 4.23(2H,m), 4.96(1H,hept,J=7Hz), 5.33(2H,m), 5.58(2H,m)
Mass(EI) m/z 422(M⁺), 404(M⁺-H₂O), 386(M⁺-2H₂O), 345(M⁺-H₂O-iC₃H₇O)
The ¹H NMR and Mass Spectra of 20-ethyl-PGA₂ isopropyl ester are shown hereinafter:
¹H NMR(CDCl₂) δ: 0.73 - 1.05(3H,m), 1.21(6H,d,J=6Hz), 1.03 - 2.70(22H,m), 3.19(1H,m), 4.05(1H,m), 4.96(1H,hept,J=6Hz), 5.36(2H,m), 5.56(2H,m), 6.14(1H,d,J=6Hz), 7.46(1H,dd,J=6Hz,J=2.5Hz)
Mass(EI) m/z 404(M⁺), 386(M⁺-H₂O), 345(M⁺-i-C₃H₂O), 327(M⁺-H₂O-i-C₃H₇O)
The ¹H NMR spectra were recorded on HITACHI R-90H (available from K.K. Hitachi Seisaku-sho) using heavy chloroform as a solvent.

Mass spectrography was measured by Mass spectrometer M-80B (available from K.K. Hitachi Seisaku-sho) with a direct inlet system at 70eV of ionizing potential. suspensions of the test drugs in a physiological saline to one eye, the intraocular pressure was measured and the intraocular pressure reduction (%) caused by each test drug was calculated. At the same time, the extent of conjunctival hyperemia was observed. The results are shown in Table 1.

The extent of conjunctival hyperemia:
- -:: none
- +-:: barely visible hyperemia
- +:: slight hyperemia
- ++:: moderate hyperemia
- +++:: severe hyperemia

**Table 1**

| Test Drug | Dose (µg/eye) | Percentage of Change of IOP | Hyperemia |
|---|---|---|---|
| 1 | 25 | 27 | + |
| 2 | 25 | 37 | ++ |
| 3 | 25 | 10 | +- |
| 4 | 25 | - | - |
| 5 | 25 | - | - |
| 6 | 100 | 46 | +++* |
| 7 | 25 | 31 | +++* |
| 8 | 10 | 32 | +++* |

| | | | |
|---|---|---|---|
| *: lid-closing and severe hyperemia are observed. | | | |

### EXAMPLE 2 (Evaluation of Intraocular Pressure and Hyperemia)

For the purpose of tonometry, Japanese White male rabbits (2.0 - 3.0 Kg) were fixed in rabbit holders. After topical anesthetization with 0.4 % oxybuprocaine hydrochloride, intraocular pressure was measured using a pheumatic applanation tonometer (manufactured by Japan Alcon). After the topical application of 50 µl of the

### Test Drug

1. 20-ethyl-PGF₂α isopropyl ester
2. 20-ethyl-PGE₂ isopropyl ester
3. 20-ethyl-PGA₂ isopropyl ester
4. 15-keto-PGF₂α
5. 15-keto-PGE₂
6. PGF₂α
7. PGF₂α isopropyl ester
8. PGE₂

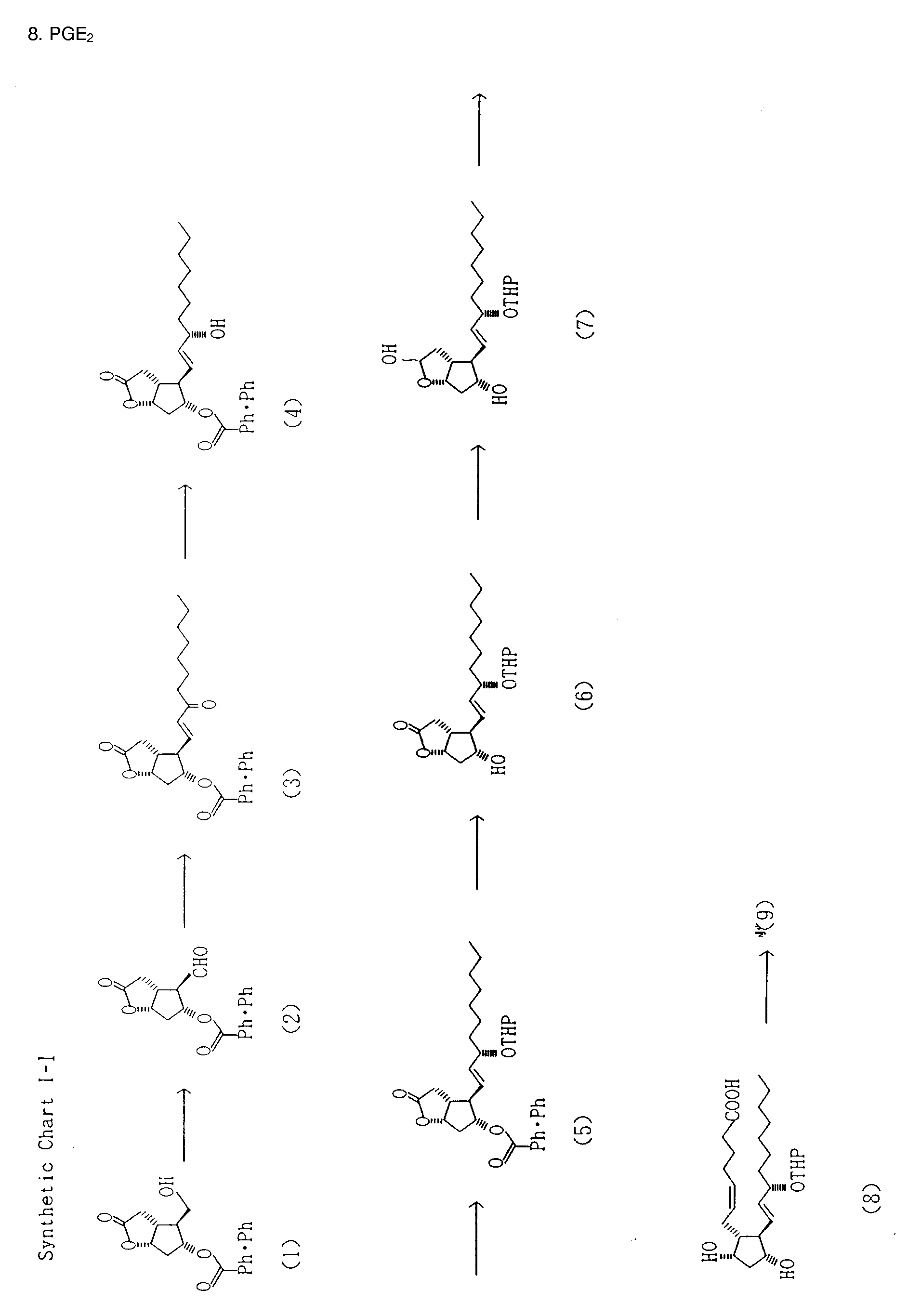

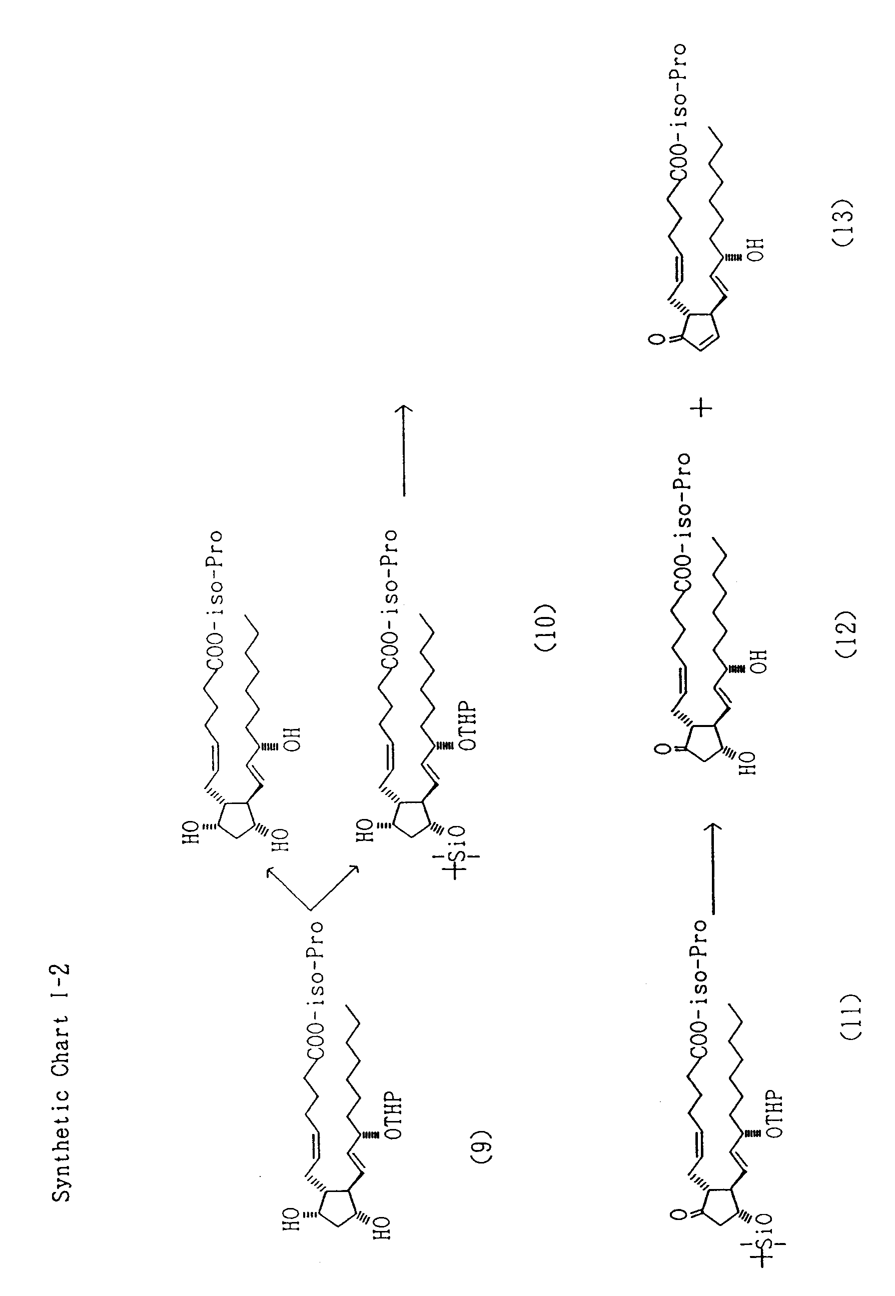

## Claims

1. The use of a composition comprising an amount of 15-hydroxy-prostaglandin of type A, E or F having one or more hydrocarbon substituent(s) at the 20-position, physiologically acceptable salts or esters thereof effective as an ocular hypotensive agent and a pharmaceutically acceptable carrier for the manufacture of a medicament for the treatment of ocular hypertension.

2. The use according to Claim 1, in which the esters are alkyl esters at the carboxyl group of terminal position of the α-chain.

3. The use according to Claim 1, in which the alkyl aster is an isopropyl ester.

4. The use according to Claim 1, in which the salts are alkaline metal, ammonium or amine salts.

5. The use according to Claim 1, in which the hydrocarbon substituent is a saturated or unsaturated C₁ - C₄ alkyl group which may have a branch.

6. The use according to Claim 1, in which the 15-hydroxy-prostaglandin is represented by a formula: 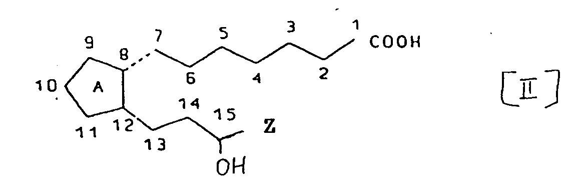 wherein A is 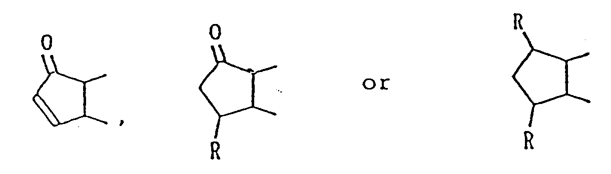 in which R is hydroxyl, hydroxyalkyl or alkyl; and Z is a C₇ - C₁₀ saturated or unsaturated aliphatic, alicyclic, aralkyl or aromatic hydrocarbon group; or physiologically acceptable salts or esters thereof.

7. The use according to Claim 6, in which the aralkyl group is phenylalkyl.

8. The use according to Claim 1, in which the 15-hydroxy-prostaglandin is represented by a formula: 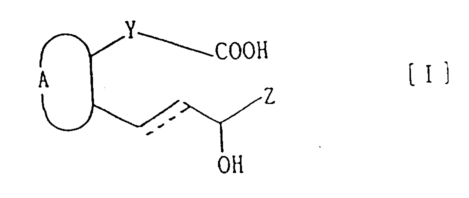 wherein A is 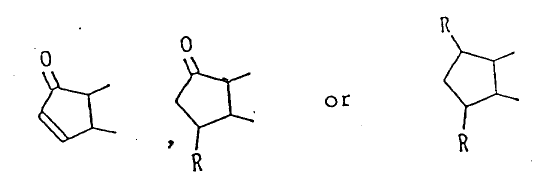 in which R is hydroxyl, hydroxyalkyl or alkyl; Y is a saturated or unsaturated C₂ - C₆ hydrocarbon chain; and Z is a C₇ - C₁₀ saturated or unsaturated aliphatic, alicyclic, aralkyl or aromatic hydrocarbon or physiologically acceptable salts derived from the general formula [I] or those having an esterified carboxyl group.

9. The use according to Claim 8, in which the aralkyl group is phenylalkyl.

10. The use of a composition comprising a 15-hydroxy-prostaglandin of type A, E, or F which has one or more hydrocarbon substituent(s) at the 20-position, physiologically acceptable salts or esters thereof effective as an ocular hypotensive agent and a pharmaceutically acceptable carrier for the manufacture of a medicament for the treatment of glaucoma.

## Patentansprüche

1. Verwendung einer Zubereitung, die eine Menge eines 15-Hydroxyprostaglandins des Typs A, E oder F, welches in 20-Position einen Kohlenwasserstoff-Subtituenten oder mehrere Kohlenwasserstoff-Substituenten besitzt, von physiologisch annehmbaren salzen oder Estern davon, die als Mittel zur Senkung des Augendrucks wirksam sind, und einen pharmazeutisch annehmbaren Träger enthält, zur Herstellung eines Arzneimittels zur Behandlung von Augenhochdruck.

2. Verwendung nach Anspruch 1, dadurch **gekennzeichnet,** daß die Ester Alkylester an der Carboxylgruppe der endständigen Position der α-Kette sind.

3. Verwendung nach Anspruch 1, dadurch **gekennzeichnet,** daß der Alkylester ein Isopropylester ist.

4. Verwendung nach Anspruch 1, dadurch **gekennzeichnet,** daß die Salze Alkalimetall-, Ammonium- oder Aminsalze sind.

5. Verwendung nach Anspruch 1, dadurch **gekennzeichnet,** daß der Kohlenwasserstoff-Substituent eine gesättigte oder ungesättigte C₁-C₄-Alkylgruppe ist, die eine Verzweigung haben kann.

6. Verwendung nach Anspruch 1, dadurch **gekennzeichnet,** daß das 15-Hydroxyprostaglandin durch die Formel: 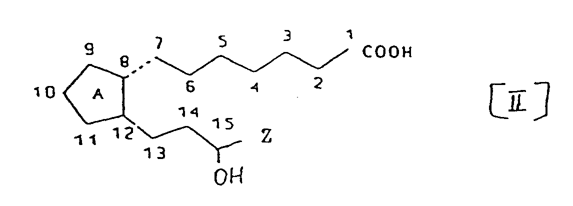 angegeben wird, worin A für 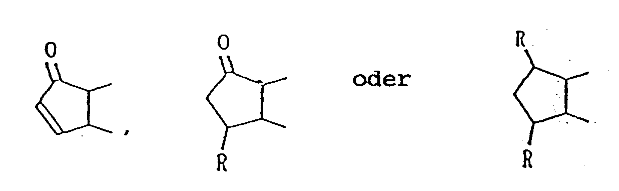 steht, wobei R eine Hydroxyl-, Hydroxyalkyl- oder Alkylgruppe ist und Z eine gesättigte oder ungesättigte aliphatische oder alicyclische C₇-C₁₀-Kohlenwasserstoffgruppe, eine Aralkyl- oder aromatische Kohlenwasserstoffgruppe ist, oder daß es ein physiologisch annehmbares Salz oder Ester davon ist.

7. Verwendung nach Anspruch 6, dadurch **gekennzeichnet,** daß die Aralkylgruppe Phenylalkyl ist.

8. Verwendung nach Anspruch 1, dadurch **gekennzeichnet,** daß das 15-Hydroxyprostaglandin durch die Formel: 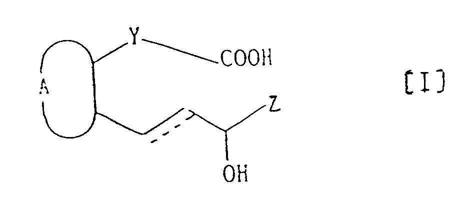 angegeben wird, worin A für 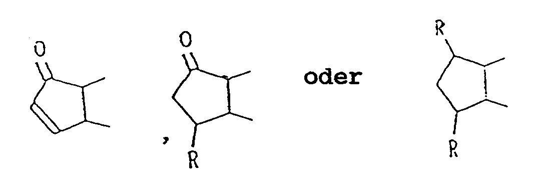 steht, wobei R eine Hydroxyl-, Hydroxyalkyl- oder Alkylgruppe ist; Y eine gesättigte oder ungesättigte C₂-C₆-Kohlenwasserstoffkette ist; und Z eine gesättigte oder ungesättigte aliphatische oder alicyclische C₇-C₁₀-Kohlenwasserstoffgruppe, eine Aralkyl- oder aromatische Kohlenwasserstoffgruppe ist, oder daß es ein physiologisch annehmbares Salz, das sich von der allgemeinen Formel (I) ableitet, oder ein solches mit einer veresterten Carboxylgruppe ist.

9. Verwendung nach Anspruch 6, dadurch **gekennzeichnet,** daß die Aralkylgruppe Phenylalkyl ist.

10. Verwendung einer Zubereitung, die ein 15-Hydroxyprostaglandin vom Typ A, E oder F, das an der 20-Position einen Kohlenwasserstoff-Substituenten oder mehrere Kohlenwasserstoff-Substituenten aufweist, physiologisch annehmbare Salze oder Ester davon, die als Mittel zur Erniedrigung des Augendrucks wirksam sind, und einen pharmazeutisch annehmbaren Träger enthält, zur Herstellung eines Arzneimittels zur Behandlung von Glaukom.

## Revendications

1. Utilisation d'une composition comprenant une quantité de 15-hydroxy-prostaglandine du type A, E, ou F ayant un ou plusieurs substituants hydrocarbonés à la position 20, leurs sels ou esters physiologiquement acceptables efficaces comme agent anti-hypertenseur oculaire et un support pharmaceutiquement acceptable pour la fabrication d'un médicament pour le traitement de l'hypertension oculaire.

2. Utilisation selon la revendication 1, dans laquelle les esters sont des esters alkyliques sur le groupe carboxyle de la position terminale de la chaîne α.

3. Utilisation selon la revendication 1, dans laquelle l'ester alkylique est un ester isopropylique.

4. Utilisation selon la revendication 1, dans laquelle les sels sont des sels de métaux alcalins, d'ammonium ou d'amine.

5. Utilisation selon la revendication 1, dans laquelle le substituant hydrocarboné est un groupe alkyle en C₁ à C₄ saturé ou insaturé qui peut avoir une ramification.

6. Utilisation selon la revendication 1, dans laquelle la 15-hydroxy-prostaglandine est représentée par la formule : 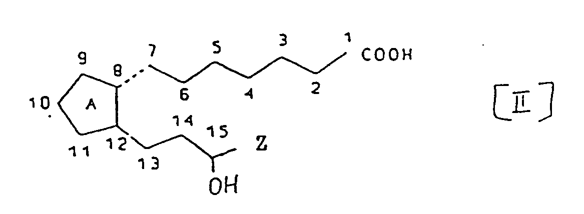 dans laquelle A est 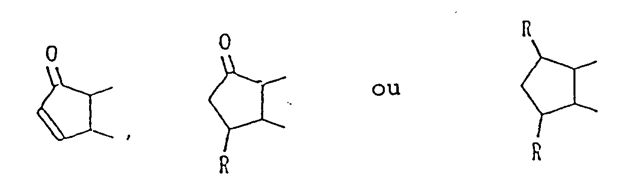 dans laquelle R est un groupe hydroxyle, hydroxyalkyle ou alkyle; et Z est un groupe hydrocarboné aliphatique, alicyclique, aralkylique saturé ou insaturé ou un groupe aromatique en C₇ à C₁₀; ou ses sels et esters physiologiquement acceptables.

7. Utilisation selon la revendication 6, dans laquelle le groupe aralkyle est un groupe phénylalkyle.

8. Utilisation selon la revendication 1, dans laquelle la 15-hydroxy-prostaglandine répond à la formule : 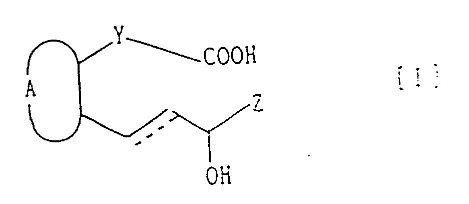 dans laquelle A est 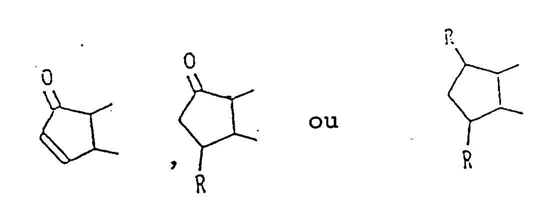 dans laquelle R est un groupe hydroxyle, hydroxyalkyle ou alkyle; Y est une chaîne hydrocarbonée en C₂ à C₆ saturée ou insaturée; et Z est un hydrocarbure aliphatique, alicyclique, aralkylique saturé ou insaturé ou un hydrocarbure aromatique en C₇ à C₁₀; ou ses sels physiologiquement acceptables dérivant de la formule générale [I], ou ceux ayant un groupe carboxyle estérifié.

9. Utilisation selon la revendication 9, dans laquelle le groupe aralkyle est un groupe phénylalkyle.

10. Utilisation d'une composition comprenant une 15-hydroxy-prostaglandine du type A, E ou F, qui porte un ou plusieurs substituants hydrocarbonés à la position 20, ses sels ou esters physiologiquement acceptables efficaces comme agent anti-hypertenseur oculaire et un support pharmaceutiquement acceptable pour la fabrication d'un médicament pour le traitement du glaucome.
